# EUROPEAN PATENT SPECIFICATION

(11) **EP 3 506 838 B1**
(45) Date of publication and mention of the grant of the patent: **13.05.2026**
(21) Application number: 17748994.5
(22) Date of filing: 21.07.2017
(51) Int. Cl.: A61B 17/04, A61B 17/06

(54) **FLEXIBLE CONSTRUCTS**
FLEXIBLE KONSTRUKTE
CONSTRUCTIONS SOUPLES

(30) Priority: 02.09.2016 US 201615255286
(43) Date of publication of application: 10.07.2019
(73) Proprietor: Arthrex, Inc, Naples, FL 34108-1945 (US)
(72) Inventor: JOLLY, Jacob A., Naples, Florida 34110 (US); PEREZ III, Arley, Naples, Florida 34105 (US); LAVIANO, Anthony A., Fort Meyers, Florida 33908 (US); OSIKA, Andrew K., Naples, Florida 34119 (US)
(74) Representative: Marks & Clerk LLP
(86) International application number: PCT/US2017/043243
(87) International publication number: WO 2018/044422

(56) References cited:
- WO-A2-2007/098212
- US-A- 5 683 417
- US-A1- 2004 032 332
- US-A1- 2014 121 700
- US-A1- 2014 257 379

## Description

### CROSS-REFERENCE TO RELATED APPLICATIONS

This disclosure claims priority U.S. Application No. 15/255,286 filed on September 2, 2016.

### BACKGROUND

This disclosure relates to surgical devices and methods for attaching body tissue, such as soft or hard tissue, to another piece of body tissue. More particularly, this disclosure is directed to flexible constructs that form loops using a barbed member to prevent the loop from loosening.

Orthopedic procedures are often performed to repair musculoskeletal injuries. For example, soft tissue may tear away from bone during vigorous exercise or sporting activities. When such tears occur, reattachment is often necessary to repair the damaged tissue. Suture anchors are one type of surgical device that has been developed to facilitate these repairs. Additional advancements in this field of technology are desired.
US2014/121700 relates to systems and methods for soft tissue to bone repairs. The splice incorporates an insert in the form of a flexible strand with at least one barb, or simply in the form of a cut length of suture or braid that is inserted into the center of the cinching loop to create a thicker portion. When the cinching loop is tightened, the insert prevents loosening of the loop, locking in the cinching loop and preventing movement of the loop. US2004/032332 A1 teaches a linkage device that can be connected with other linkage devices and to itself, the linkage device comprising a strip having a first end and a second end, a closure hub attached to the first end of the strip and having an opening to receive a second end and a locking mechanism within the closure hub, a connecting surface protruding from the strip operable to secure the second end within a closure device, and a tampering detection device connected to the linkage device.
US5683417 A relates to a suture for use in endoscopic surgery includes a distal end needle, a preferably braided suture body, and a bulbous proximal end. The method includes stitching the tissue, then passing the needle/distal end through the bulbous proximal end, where the body of the suture is held frictionally or adhesively under proper tension.
WO2007/098212 A2 relates to endoscopic suturing devices for transgastric flexible endoscopy procedures. In a first aspect, the endoscopic suturing device includes a hollow helical needle carrying an elongated suture thread with a suture anchor on the distal end of the suture thread. The suture anchor has a low-profile insertion position and an expanded anchoring position. A suture lock is provided for attachment at the proximal end of the suture thread. In another aspect, the suture device includes a microcarabiner and suture lock. The suture device may take the form of a conventional suture thread or a zip-tie fastener. In an alternate configuration, the microcarabiner can be combined with a tissue anchor. One or more such tissue anchors can be used to form a running suture, purse string suture or other suture configurations.
US2014/257379 A1 relates to a device including a self-locking suture including both a braided section and a monofilament section. The monofilament includes a single strand. The braided section includes three or more strands that are intertwined or woven together. The terminal end of the braid is compressible to take on a radially expanded configuration. The braided structure is temporarily fixable in the open configuration providing a wide berth opening. A needle at the terminal end of the monofilament is insertable through the wide berth opening and pushed out through the back wall of the expanded braid, after which the braid can be pulled on to collapse it down onto the barbed monofilament. The construct may then be tightened onto the tissues to be joined, and the one-way locking mechanism between the braided trap and the directionally biased suture automatically engages.

### SUMMARY

Aspects of the present invention are set out in the appended claims. This disclosure relates to surgical fixation devices and methods. The surgical fixation devices include a flexible construct that forms an adjustable loop that can be tightened but restricts movement that would loosen the adjustable loop. The surgical fixation devices can be used in various tissue reconstruction procedures, including but not limited to, ACL and PCL reconstructions and capsular closure.

A flexible construct, not within the scope of the appended claims includes, *inter alia,* a sheath and a suture connected to the sheath to form an adjustable loop. The suture includes a plurality of barbs that permit movement of the suture in a first direction relative to the sheath and restrict movement in a second direction relative to the sheath.

A surgical method, not within the scope of the appended claims includes, *inter alia,* attaching a first piece of tissue to a second piece of tissue using a tissue closure assembly. The tissue closure assembly includes a sheath and a barbed suture. The barbed suture includes a plurality of barbs that permit movement of the barbed suture in a first direction relative to the sheath and restrict movement in a second direction relative to the sheath.

A flexible construct is provided according to appended claim 1.

A surgical method, not within the scope of the appended claims, includes, *inter alia,* passing a suture through a first splice in a first direction and restricting movement of the suture through the first splice in a second direction with a barbed device.

### BRIEF DESCRIPTION OF THE DRAWINGS

Figure 1 illustrates a flexible construct, such as a tissue closure assembly.
Figure 2A illustrates a cross-sectional view of a sheath of the flexible construct of Figure 1.
Figure 2B schematically illustrates an example for inserting a suture through a sheath of the flexible construct of Figure 1.
Figure 3A illustrates the flexible construct of Figure 1 approximating tissue.
Figure 3B illustrates a flexible construct approximating tissue.
Figure 4 illustrates a flexible construct according to an embodiment.
Figure 5 illustrates a button used in connection with the flexible construct of Figure 4.
Figure 6 illustrates a cross-sectional view taken through splices in the flexible construct of Figure 4.
Figure 7 illustrates a flexible construct according to yet another embodiment.
Figure 8 illustrates a cross-sectional view taken through a slice of the flexible construct of Figure 7.
Figure 9 illustrates a flexible construct according to a further example.

### DETAILED DESCRIPTION

This disclosure relates to surgical fixation devices and methods. Surgical fixation devices include a flexible construct that forms an adjustable loop that can be tightened but restrict movement that would loosen the adjustable loop. The surgical fixation devices can be used in various tissue reconstruction procedures, including but not limited to, ACL and PCL reconstructions and capsular closure.

A tissue closure assembly, not within the scope of the claims includes, *inter alia,* a sheath and a suture connected to the sheath to form an adjustable loop. The suture includes a plurality of barbs that permit movement of the suture in a first direction relative to the sheath and restrict movement in a second direction relative to the sheath.

In a further example, a sheath includes a first sheath end and a second sheath end.

In a further example, a suture includes a first suture end and a second suture end. A first suture end is attached to a first sheath end.

In a further example, a plurality of barbs is directed towards a first suture end.

In a further example, a first suture end is fixedly received within a sheath at a first sheath end.

In a further example, a suture is fixedly attached to a sheath with an adhesive.

In a further example, a second suture end is received in a second sheath end.

In a further example, an exit hole is located in a sheath between a first sheath end and a second sheath end.

In a further example, a second end of a suture is configured to be received within an exit hole.

In a further example, a sheath is tubular in shape and is made of a fibrous material.

A surgical method, not within the scope of the appended claims includes, *inter alia,* attaching a first piece of tissue to a second piece of tissue using a tissue closure assembly. The tissue closure assembly includes a sheath and a barbed suture. The barbed suture includes a plurality of barbs that permit movement of the barbed suture in a first direction relative to the sheath and restrict movement in a second direction relative to the sheath.

In a further example, a sheath includes a first sheath end and a second sheath end.

In a further example, a barbed suture includes a first suture end and a second suture end. A first suture end is attached to a first sheath end.

In a further example of the disclosed methods, an attaching step includes inserting a second suture end into the second sheath end and engaging a portion of a sheath with a plurality of barbs to prevent a second suture end from separating from the sheath.

In a further example of the disclosed methods, an attaching step includes passing a second suture end through an exit hole in a side of a sheath.

In a further example of the disclosed methods, an attaching step includes surrounding a first piece of tissue and a second piece of tissue with a barbed suture and a sheath.

In a further example, a first piece of tissue and a second piece of tissue are portions of the same tissue.

A flexible construct according to another exemplary aspect of the present disclosure includes, *inter alia,* a suture and a first splice in the suture. A barbed device is located at least partially within the splice and is configured to reinforce the splice.

In a further embodiment, a first splice includes a first splice entrance and a first splice exit. A first portion of a barbed device is located between a first splice entrance and a first splice exit.

In a further embodiment, a barbed device includes a second portion located within a suture spaced from a first splice.

A first plurality of barbs and a second plurality of barbs are directed towards a mid-portion of a barbed device.

In a further embodiment, a first splice forms a first eyelet splice.

In a further embodiment, there are a second splice and a button. A second splice forms a second splice eyelet. The first eyelet splice and the second eyelet splice form an adjustable loop with the button.

A surgical method, not within the scope of the appended claims, includes, *inter alia,* passing a suture through a first splice in a first direction and restricting movement of the suture through the first splice in a second direction with a barbed device.

In a further example, a barbed device is located within an interior of the suture and includes a first plurality of barbs directed in a first direction and a second plurality of barbs directed in a second opposite direction.

In a further example, a first plurality of barbs and a second plurality of barbs are directed towards a mid-portion of the barbed device.

In a further example, a suture is passed through a second splice in a third direction and movement is restricted of the suture through the second splice in a fourth direction.

In a further example, a suture forms an adjustable loop, and a barbed device prevents the adjustable loop from expanding in size.

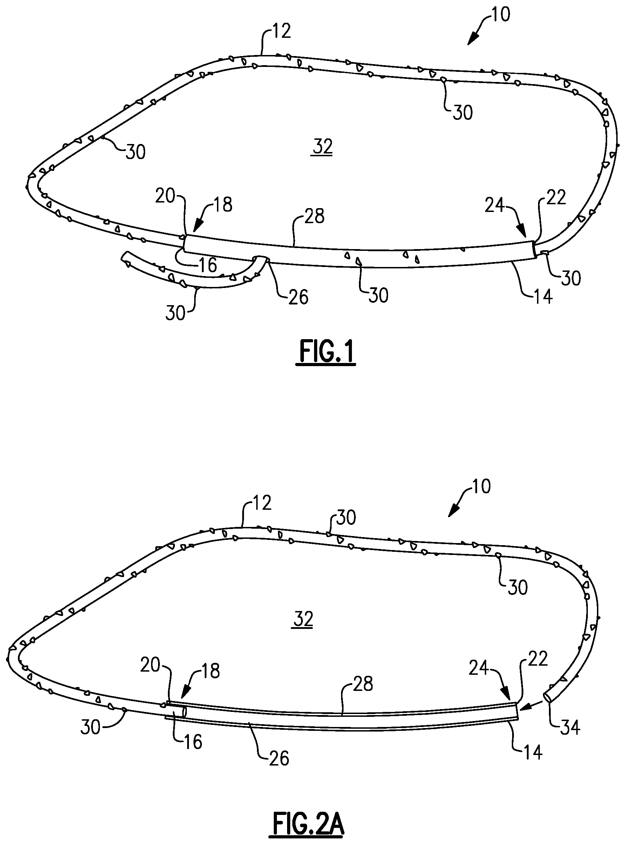

Figure 1 illustrates a tissue closure assembly 10. The tissue closure assembly 10 includes a suture 12 and a sheath 14. In the illustrated embodiment, a first end 16 of the suture 12 is received within a first end portion 18 of the sheath 14. The sheath 14 includes an elongated tubular shaped wall 28 that defines a first opening 20 in the first end portion 18 and a second opening 22 in a second end portion 24 of the sheath 14. The sheath 14 may be made of a fibrous material, a polymer, or a composite material as non-limiting examples. An exit hole 26 is located between the first opening 20 and the second opening 22. The exit hole 26 extends through the tubular shaped wall 28 of the sheath 14.

As shown in Figure 2A, the first end 16 of the suture 12 includes an outer diameter that fits within an inner diameter of the first opening 20 of the sheath 14. The first end 16 of the suture 12 is attached to the sheath 14 with an adhesive or other bonding agent or process. The suture 12 includes multiple barbs 30 projecting outward an outer surface of the suture 12. The suture 12 may therefore be referred to as a barbed suture. In the illustrated embodiment, all of the barbs 30 are directed towards the first end 16 of the suture 12.

The suture 12 and the sheath 14 form a loop 32 when a second end 34 of the suture 12 is inserted into the second opening 22 in the sheath 14. Since the barbs 30 are oriented towards the first end 16 of the suture 12, the second end 34 of the suture 12 is able to move through the sheath 14 in a direction from the second opening 22 towards the first opening 20 but is prevented from moving in an opposite direction from the first opening 20 towards the second opening 22 by the barbs 30 engaging the sheath 14. A diameter of the loop 32 is reduced as the suture 12 is fed into the sheath 14. The loop 32 may be further tightened or constricted by pulling the second end 34 of the suture 12 until it has been inserted through the exit hole 26 in the sheath 14.

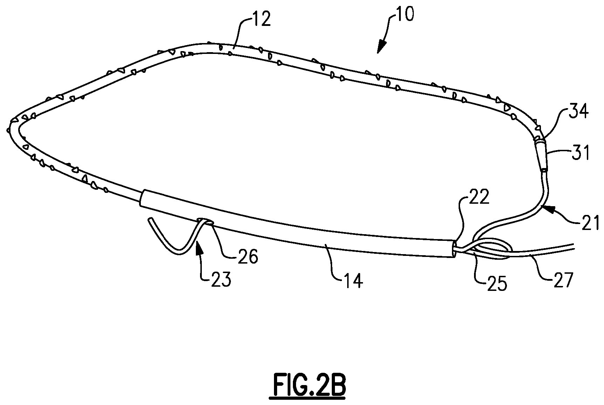

Figure 2B illustrates an example for inserting the suture 12 into the sheath 14 of the tissue closure assembly 10. The exemplary insertion method uses a leader suture 21 and a loading suture 23. The leader suture 21 may be attached to the second end 34 of the suture 12. In an embodiment, the leader suture 21 includes a cap 31 that is insertable over the second end 34 of the suture 12 to connect the leader suture 21 to the suture 12. The leader suture 21 aids in passing the suture 12 through tissue and subsequently through the sheath 14. The loading suture 23 is inserted through the exit hole 26 of the sheath and the second opening 22 of the sheath 14. The loading suture 23 may include a loop 25 configured to receive a tail 27 of the leader suture 21. The suture 12 can be shuttled into the sheath 14 by inserting the tail 27 through the loop 25 and then pulling the loading suture 23 through the sheath 14 by pulling the portion of the loading suture 23 that extends out of the exit hole 26.

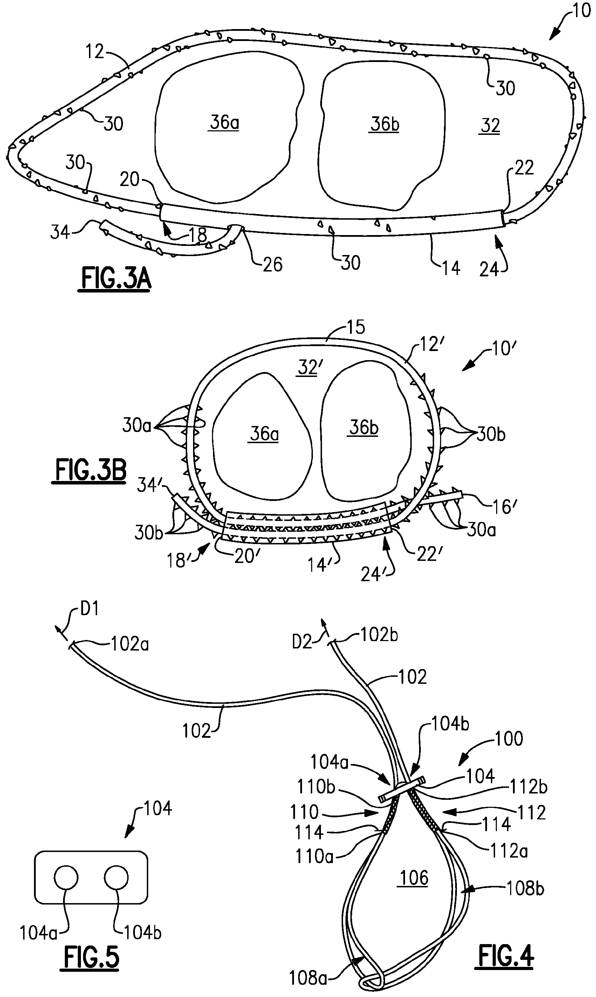

Figure 3A illustrates an exemplary use of the tissue closure assembly 10. As shown in Figure 3A, the tissue closure assembly 10 is used for approximating a first piece of tissue 36a relative to a second piece of tissue 36b. The first and second pieces of tissue 36a, 36b may be pieces of the same tissue or separate pieces of tissue. The tissue closure assembly 10 may also be used to approximate tissue in a capsular closure technique, for example.

The tissue closure assembly 10 of this disclosure provides an improved tissue closure device and technique. In particular, the barbed suture 12 reduces the complexity of forming a tissue securing loop by reducing the need to tie knots to form a loop which can be difficult and time consuming during arthroscopic procedures. Alternatively, the suture 12 and sheath 14 of the tissue closure assembly 10 could penetrate and pass through either the first and second pieces of tissue 36a, 36b.

Figure 3B illustrates a tissue closure assembly 10' according to another example. The tissue closure assembly 10' is similar to the tissue closure assembly 10 except where shown in the Figures or described below. The tissue closure assembly 10' includes a suture 12' and a sheath 14'. In the illustrated embodiment, the suture 12' includes a first end 16' and a second end 34.' The sheath 14' includes a first end portion 18' having a first opening 20' and a second end portion 24' having a second opening 22'.

The suture 12' includes a first plurality of barbs 30a and a second plurality of barbs 30b. The first plurality of barbs 30a is directed towards the second end 34' or a mid-portion 15 of the suture 12' and the second plurality of barbs 30b is directed towards the first end 16' or the mid-portion 15 of the suture 12'.

The first end 16' of the suture 12' is fed through the first opening 20' in the sheath 14' until the first end 16' passes out of the second opening 22' in the sheath 14'. Similarly, the second end 34' of the suture 12' is fed through the second end 22' of the sheath 14' until the second end 34' passes out of the first opening 20' of the sheath 14'. A size of a loop 32' defined by the suture 12' and the sheath 14' can be reduced by pulling on the first and second ends 16', 34' of the suture 12'. The loop 32' is prevented from expanding in size by the first plurality of barbs 30a engaging second plurality of barbs 30b. Moreover, the first and second plurality of barbs 30a, 30b engage an interior surface of the sheath 14' to further prevent the loop 32' from expanding.

The tissue closure assembly 10' may also be used for approximating the first piece of tissue 36a relative to the second piece of tissue 36b, such as in a capsular closure technique, for example.

Figure 4 illustrates a button/loop construct 100 that may be configured as a flexible construct according to an embodiment of the present invention. The construct 100 includes a flexible strand of suture 102 and a button 104 that form an adjustable loop 106. In one example, the construct 100 can be used to connect tissue to bone for passing the button 104 through a bone tunnel in a first position generally parallel to the bone tunnel and then flipping the button 104 to a second position generally perpendicular to the bone tunnel. The adjustable loop 106 can be tightened by pulling a first end 102a and a second end 102b of the strand 102 in the directions D1 and D2, respectively. However, as will be described further below, the construct 100 includes a barbed device 114 that prevents the adjustable loop 106 from expanding and loosening once tightened by pulling the first and second ends 102a, 102b.

As shown in Figures 4 and 5, the construct 100 forms the adjustable loop 106 by threading the first end 102a of the strand 102 into a first opening 104a in the button 104 and then threading the second end 102b of the strand 102 through a second opening 104b in the button 104. Once the first and second ends 102a, 102b of the strand 102 have passed through the button 104, the first end 102a of the strand 102 is spliced back into the strand 102 at a first splice 110 to form a first eyelet splice 108a. The first end 102a may be spliced back into the strand with the assistance of a needle or other device. The first end 102a enters the first splice 110 at a first splice entrance 110a and exits the strand 102 at a first splice exit 110b. When the first end 102a exits the first splice exit 110b, the first end 102a is threaded back through the first opening 104a in the button 104. In the illustrated embodiment, the first splice exit 110b is located in close proximity to the first opening 104a in the button 104.

A second eyelet splice 108b is formed in a similar manner to the first eyelet splice 108a described above. After the second end 102b of the strand 102 is passed through the second opening 104b in the button 104, the second end 102b is passed through the first eyelet splice 108a to link the first eyelet splice 108a to the second eyelet splice 108b to form the adjustable loop 106. The second end 102b is then threaded into a second splice 112 in the strand 102. The second end 102b may be spliced back into the strand with the assistance of a needle or other device. The second end 102b enters the strand 102 at a second splice entrance 112a and exits the strand 102 at a second splice exit 112b. In the illustrated embodiment, the second splice exit 112b is located adjacent the second opening 104b in the button 104. The second splice exit 112b is space from the first splice exit 110b by a distance similar to the spacing between the first opening 104a and the second opening 104b in the button 104.

Once the first and second eyelet splices 108a, 108b have formed the adjustable loop 106, a size of the adjustable loop 106 can be reduced by pulling on the first and second ends 102a, 102b in the directions D1, D2, respectively. Once a desired size for the adjustable loop 106 is reached, the first end 102a of the strand 102 is fed through the second opening 104b in the button 104, and the second end 102b of the strand 102 is fed through the first opening 104a in the button 104. By feeding the first and second ends 102a, 102b through the button 104, the strand 102 is prevented from moving relative to the button 104 such that the size of the adjustable loop 106 will not increase.

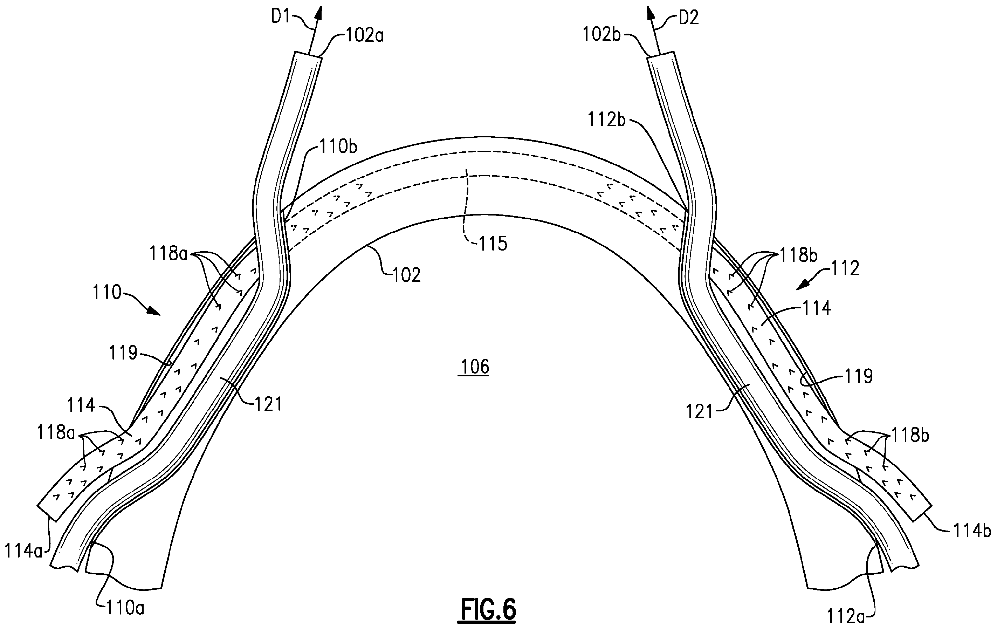

In addition to simply threading the first and second suture ends 102a, 102b back through the button 104, the construct 100 has additional fixation to secure the strand 102 from moving and increasing the size of the adjustable loop 106. This is accomplished with the use of the barbed device 114 as shown in Figure 6.

Figure 6 illustrates a cross-sectional view taken through the first and second splices 110, 112 of the construct 100 of Figure 4 (shown without the button 104 for clarity). As shown in Figure 6, the barbed device 114 extends between the first splice 110 and the second splice 112. In the illustrated embodiment, the barbed device 114 includes a first plurality of barbs 118a and a second plurality of barbs 118b. The first plurality of barbs 118a is located adjacent a first end 114a of the barbed device 114. The first plurality of barbs 118a is also at least partially located within an area of the first splice 110 and is oriented towards a mid-portion 115 of the barbed device 114. The second plurality of barbs 118b is located adjacent a second end 114b of the barbed device 114. The second plurality of barbs 118b is also at least partially located within an area of the second splice 112 and is oriented towards the mid-portion 115 of the barbed device 114. Since the first plurality of barbs 118a is oriented towards the mid-portion 115 of the barbed device 114, the strand 102 is able to pass through the first splice 110 when a force in the direction D1 is applied to the first end 102a. Similarly, because the second plurality of barbs 118b is oriented towards the mid-portion 115 of the barbed device 114, the strand 102 is able to pass through the second splice 112 when a force is applied in the direction D2.

Moreover, the barbed device 114 prevents the adjustable loop 106 from expanding and the first and second ends 102a, 102b from moving in a direction opposite D1, D2, respectively. In order to prevent expansion of the adjustable loop 106, the first plurality of barbs 118a engages an interior portion 119 of the strand 102 adjacent the first splice 110, and the second plurality of barbs 118b engages an exterior portion 121 of the strand 102 adjacent the second splice 112 when a force is applied to the adjustable loop 106 that would expand its size. Similarly, the second plurality of barbs 118b also engages the interior portion 119 of the strand 102 adjacent the second splice 112, and the first plurality of barbs 118a engages an exterior portion of the strand 102 adjacent the first splice 110 when a force is applied to the adjustable loop 106 that would expand its size.

In an embodiment, the barbed device 114 is a barbed suture. However, other devices may also be suitable for use as the barbed device 114 for reinforcing a spliced portion of a flexible construct.

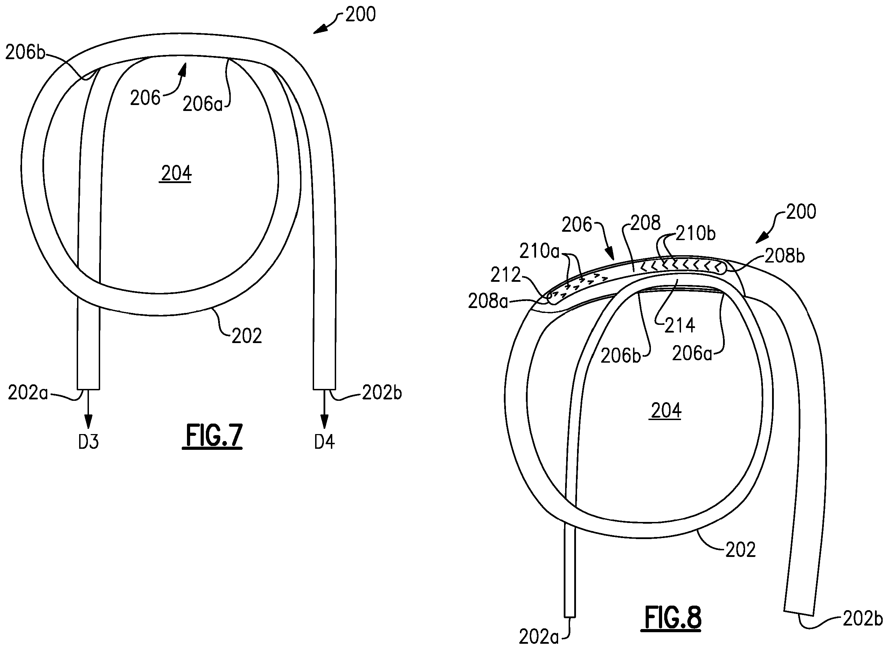

Figure 7 illustrates as a loop construct 200 a flexible construct according to yet another embodiment. The construct 200 includes a strand 202 that forms an adjustable loop 204. The adjustable loop 204 is formed by splicing the strand 202 at a first splice 206. The first splice 206 is formed by feeding a first end 202a of the strand 202 back through a portion of the strand 202 as shown in Figures 7 and 8.

The adjustable loop 204 can be adjusted by pulling on the first end 202a and a second end 202b of the strand 202 in the directions D3 and D4, respectively. However, as will be described further below, the construct 200 includes a barbed device 208 that prevents the adjustable loop 204 from expanding and loosening once tightened by pulling the first and second ends 202a, 202b.

As shown in the cross-sectional view of the first splice 206 of the construct 200 in Figure 8, a portion of the strand 202 and the barbed device 208 are located adjacent the first splice 206. The barbed device 208 includes a first distal end 208a and a second distal end 208b. The first distal end 208a of the barbed device 208 is closer to the first end 202a of the strand 202, and the second distal end 208b of the barbed device 208 is closer to the second end 202b of the strand 202. The barbed device 208 includes a first plurality of barbs 210a and a second plurality of barbs 210b. The first plurality of barbs 210a is oriented in a direction towards the second end 208b of the barbed device 208, and the second plurality of barbs 210b is oriented towards the first end 208a of the barbed device 208.

When the construct 200 is tightened by pulling on the first and second ends 202a, 202b of the strand 202 in the directions D3, D4, respectively, the portion of the strand 202 within the first splice 206 is able to slide freely in a direction from a first splice entrance 206a to a first splice exit 206b. When a force is applied to the construct 200 that would expand the adjustable loop 204, the first plurality of barbs 208a engages an interior surface 212 of the of the strand 202, and the second plurality of barbs 208b engages an exterior surface 214 of the strand 202 adjacent the first splice 206.

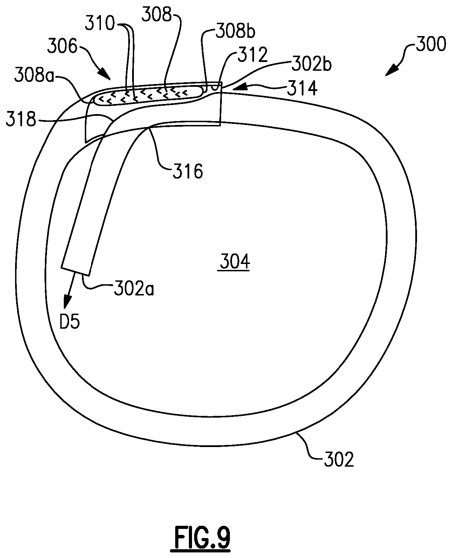

Figure 9 illustrates as a loop construct 300 a flexible construct according to a further example. The construct 300 includes a strand 302 that forms an adjustable loop 304. The adjustable loop 304 is formed by splicing the strand 302 at a first splice 306. The first splice 306 is formed by feeding a first end 302a of the strand 302 into an opening 314 defined by a second end 302b of the strand 302 and out a first splice exit 316.

The adjustable loop 304 can be adjusted by pulling on the first end 302a of the strand 302 in a direction D5. However, as will be described further below, the construct 300 includes a barbed device 308 that prevents the adjustable loop 304 from expanding and loosening once tightened by pulling the first end 302a.

As shown in the cross-sectional view of the first splice 306 of the construct 300 in Figure 9, a portion of the strand 302 and the barbed device 308 are located adjacent the first splice 306. The barbed device 308 includes a first distal end 308a and a second distal end 308b. The first distal end 308a of the barbed device 308 is closer to the first end 302a of the strand 302 and the second distal end 308b of the barbed device 308 is closer to the second end 302b of the strand 302. The barbed device 308 includes a first plurality of barbs 310 oriented in a direction towards the first end 308a of the barbed device 308. The barbed device 308 may be secured to an interior of the strand 302 by an adhesive or other similar method.

When the construct 300 is tightened by pulling on the first ends 302a of the strand 302 in the directions D5, the portion of the strand 302 within the first splice 306 is able to slide freely in a direction from the opening 314 to the first splice exit 316. When a force is applied to the construct 300 that would expand the adjustable loop 304, the first plurality of barbs 310 engages an exterior surface 318 of the strand 302 adjacent the first splice 306.

The loop constructs 100, 200, and 300 of this disclosure provide an improved adjustable loop device and technique. The barbs on the barbed device prevent expansion of the adjustable loop once the loop has been tightened. This provides a stronger attachment with a reduction in the loop loosening after placement in the body.

In another embodiment, the barbed devices 114, 208, 308 of this disclosure can be utilized within any spliced construct. The barbed devices 114, 208, 308 can be positioned within the spliced portion of the construct for reinforcing a locking mechanism of the spliced portion.

It should be understood that like reference numerals identify corresponding or similar elements throughout the several drawings.

## Claims

1. A flexible construct (100, 200), comprising:
a suture (102, 202);
at least one splice (110, 206) in the suture (102, 202); and
a barbed device (114, 208) located at least partially within the splice (110, 206) and configured to reinforce the splice (110, 206),
**characterized in that** the barbed device (114, 208) includes a first plurality of barbs (118a, 210a) and a second plurality of barbs (118b, 210b), and wherein the first plurality of barbs (118a, 210a) and the second plurality of barbs (118b, 210b) are directed towards a mid-portion (115) of the barbed device (114, 208).

2. The flexible construct (100, 200) of claim 1, wherein the at least one splice (110, 206) includes a splice entrance (110a, 206a) and a splice exit (110b, 206b); and a portion of the barbed device (114) is located between the splice entrance (110a, 206a) and the splice exit (110b, 206b).

3. The flexible construct (100, 200) of claim 2, wherein the barbed device (114, 208) includes a second portion located within the suture (102, 202) spaced from the at least one splice (110, 206).

4. The flexible construct (100, 200) of claim 1, wherein the at least one splice (110) forms an eyelet splice (108a).

5. The flexible construct (100) of claim 4, wherein the at least one splice (110) comprises a second splice (112) and a button (104) wherein the second splice (112) forms a second eyelet splice (108b) and the first eyelet splice (108a) and the second eyelet splice (108b) form an adjustable loop (106) with the button (104).

## Patentansprüche

1. Flexibles Konstrukt (100, 200), umfassend: ein Nahtmaterial (102, 202);
mindestens einen Spleiß (110, 206) in dem Nahtmaterial (102, 202); und
eine mit Widerhaken versehene Vorrichtung (114, 208), die sich zumindest teilweise innerhalb des Spleißes (110, 206) befindet und konfiguriert ist, den Spleiß (110, 206) zu verstärken,
**dadurch gekennzeichnet, dass** die mit Widerhaken versehene Vorrichtung (114, 208) eine erste Vielzahl von Widerhaken (118a, 210a) und eine zweite Vielzahl von Widerhaken (118b, 210b) umfasst, und wobei die erste Vielzahl von Widerhaken (118a, 210a) und die zweite Vielzahl von Widerhaken (118b, 210b) auf einen mittleren Abschnitt (115) der mit Widerhaken versehenen Vorrichtung (114, 208) gerichtet sind.

2. Flexibles Konstrukt (100, 200) nach Anspruch 1, wobei der mindestens eine Spleiß (110, 206) einen Spleißeingang (110a, 206a) und einen Spleißausgang (110b, 206b) umfasst; und ein Abschnitt der mit Widerhaken versehenen Vorrichtung (114) zwischen dem Spleißeingang (110a, 206a) und dem Spleißausgang (110b, 206b) angeordnet ist.

3. Flexibles Konstrukt (100, 200) nach Anspruch 2, wobei die mit Widerhaken versehene Vorrichtung (114, 208) einen zweiten Abschnitt aufweist, der innerhalb des Nahtmaterials (102, 202) angeordnet ist und von dem mindestens einen Spleiß (110, 206) beabstandet ist.

4. Flexibles Konstrukt (100, 200) nach Anspruch 1, wobei der mindestens eine Spleiß (110) einen Ösenspleiß (108a) bildet.

5. Flexibles Konstrukt (100) nach Anspruch 4, wobei der mindestens eine Spleiß (110) einen zweiten Spleiß (112) und einen Knopf (104) umfasst, wobei der zweite Spleiß (112) einen zweiten Ösenspleiß (108b) bildet und der erste Ösenspleiß (108a) und der zweite Ösenspleiß (108b) eine einstellbare Schlaufe (106) mit dem Knopf (104) bilden.

## Revendications

1. Construction flexible (100, 200), comprenant : une suture (102, 202) ;
l'au moins une épissure (110, 206) dans la suture (102, 202) ; et
un dispositif barbelé (114, 208) situé au moins partiellement à l'intérieur de l'épissure (110, 206) et configuré pour renforcer l'épissure (110, 206),
**caractérisée en ce que** le dispositif barbelé (114, 208) comprend une première pluralité de barbillons (118a, 210a) et une deuxième pluralité de barbillons (118b, 210b), et dans lequel la première pluralité de barbillons (118a, 210a) et la deuxième pluralité de barbillons (118b, 210b) sont dirigées vers une partie médiane (115) du dispositif barbelé (114, 208).

2. Construction flexible (100, 200) selon la revendication 1, dans laquelle au moins une épissure (110, 206) comprend une entrée d'épissure (110a, 206a) et une sortie d'épissure (110b, 206b) ; et une partie du dispositif barbelé (114) est située entre l'entrée d'épissure (110a, 206a) et la sortie d'épissure (110b, 206b).

3. Construction flexible (100, 200) selon la revendication 2, dans laquelle le dispositif barbelé (114, 208) comprend une deuxième partie située à l'intérieur de la suture (102, 202) espacée d'au moins une épissure (110, 206).

4. Construction flexible (100, 200) selon la revendication 1, dans laquelle l'au moins une épissure (110) forme une épissure en œillet (108a).

5. Construction flexible (100) selon la revendication 4, dans laquelle l'au moins une épissure (110) comprend une deuxième épissure (112) et un bouton (104), dans laquelle la deuxième épissure (112) forme une deuxième épissure d'œillet (108b) et la première épissure d'œillet (108a) et la deuxième épissure d'œillet (108b) forment une boucle réglable (106) avec le bouton (104).
